Europäisches Patentamt

European Patent Office

Office européen des brevets

(19)

(11) Publication number: 0 184 896
A1

# (12) EUROPEAN PATENT APPLICATION

(21) Application number: 85307403.7

(22) Date of filing: 15.10.85

(51) Int. Cl.⁴: **C 07 C 19/08, C 07 C 17/20**

(30) Priority: 25.10.84 GB 8427033

(43) Date of publication of application: 18.06.86
Bulletin 86/25

(84) Designated Contracting States: **DE FR GB IT**

(71) Applicant: **IMPERIAL CHEMICAL INDUSTRIES PLC, Imperial Chemical House Millbank, London SW1P 3JF (GB)**

(72) Inventor: **Burgess, Leslie, 1 Poolside Road, Runcorn Cheshire (GB)**
Inventor: **Maling, Guy Quentin, Church Croft Horton Tilston Cape, Malpas Cheshire (GB)**
Inventor: **Speedy, James, 19 Coniston Drive, Frodsham Nr Warrington (GB)**

(74) Representative: **Stephenson, Kenneth et al, Imperial Chemical Industries PLC Legal Department: Patents PO Box 6 Bessemer Road, Welwyn Garden City Herts, AL7 1HD (GB)**

(54) Process for the preparation of bromotrifluoromethane.

(57) A method for the preparation of bromotrifluoromethane which comprises contacting gaseous dibromodifluoromethane with a fluorination catalyst at a temperature in the range 100-45°C.

TITLE MODIFIED
see front page
0184896
QM. 33280 EP

CHEMICAL PROCESS

This invention relates to a chemical process and more particularly to a method for the preparation of bromotrifluoromethane.

Bromotrifluoromethane is a known chemical compound useful as a fire-extinguishing agent and as a refrigerant. It is usually manufactured by the bromination of trifluoromethane as described, for example, in German Patent 1 155 104. This method suffers from the disadvantages that trifluoromethane requires special storage apparatus because of its very low boiling point and that special materials of construction are required for the reactor in order to avoid severe corrosion by the bromine at the high reaction temperature (650-800°C). When chlorine is included in the reaction mixture to lower the bromination temperature as described in United States Patent 2658086, a substantial amount of chlorotrifluoromethane is produced as by-product reducing the yield of bromotrifluoromethane to 36.6 mole per cent.

Another method that has been proposed in German Offenlegungsschrift 1913405 is to contact dibromodifluoromethane in a solvent with aluminium chloride or aluminium bromide at a temperature between -20 and 120°C, the dibromodifluoromethane disproportionating to form bromotrifluoromethane and tetrabromomethane. The temperature conditions are so selected that the dibromodifluoromethane is present as a liquid or completely dissolved in the solvent. In the absence of sufficient solvent to prevent crystallisation of the tetrabromomethane, it is said, the reaction stops.

It has now been found that bromotrifluoromethane can be prepared in good yield by a catalytic process using gaseous dibromodifluoromethane.

Thus, according to the invention, there is provided a method for the preparation of bromotrifluoromethane which comprises contacting gaseous dibromodifluoromethane with a fluorination catalyst at a temperature in the range 100-450°C.

The dibromodifluoromethane used in the method of the invention may be substantially pure or it may be a commercial grade containing bromochlorodifluoromethane. An inert gaseous diluent, for example nitrogen or helium, may be present if desired.

Fluorination catalysts are well known in the art and include the following:

1) Iron on carbon

2) Mixed oxide of aluminium, chromium and magnesium.

3) Chromium oxide catalysts as described in our United Kingdom Patent specification No. 1307224, the chromium oxide optionally having been subjected to a prefluorination treatment with the hydrogen fluoride before use. Also chromium fluoride and chromium oxyfluoride. Any of these may be used on a carbon support.

4) Alumina that has been treated with hydrogen fluoride.

5) Aluminium fluoride alone or with alumina.

6) Potassium, rubidium or cerium fluoride alone or with alumina or aluminium fluoride.

7) Chromium, cobalt, nickel or thorium oxide or fluoride optionally on a support such as graphite.

8) Thorium, zirconium or palladium fluoride on carbon.

9) Magnesium or tungsten fluoride.

10) Active carbon

Aluminium fluoride should be given a pre-activation treatment by heating in a stream of dibromodifluoromethane, with diluent if desired, to about 350°C. Thereafter, the reaction may be carried out at the desired temperature.

The catalyst may be employed in the form of a fixed or fluidised bed of appropriate size.

The method of the invention may be conveniently performed at normal atmospheric pressure but higher or lower pressures may be used if desired.

The reaction taking place in the method of the invention is believed to be disproportionation of the dibromodifluoromethane forming bromotrifluoromethane and tetrabromomethane. The yield of tetrabromomethane may be reduced with a corresponding increase in bromotrifluoromethane production by including hydrogen fluoride in the gas feed.

Thus, a preferred embodiment of the invention comprises contacting a gaseous mixture of dibromodifluoromethane and hydrogen fluoride with a catalyst at a temperature in the range 100-400°C.

When hydrogen fluoride is present in the gas feed, the molar ratio of hydrogen fluoride to dibromodifluoromethane is suitably in the range 0.1-5:1, a molar ratio of 1:1 being particularly suitable.

Although the method of the invention may be carried out in a batchwise manner, it is preferably carried out continuously. Conventional means may be used to recover the reaction product and to isolate the bromotrifluoromethane from any by-products.

-4-

The invention is illustrated but not limited by the following Examples:

Example 1

A tubular reactor was charged with 800cm$^3$ of dry aluminium fluoride (KAF 1000) specially prepared for use in fluidised beds and having a mean particle size of 150 μm. A helium gas flow of 200cm$^3$/min was maintained during charging to assist fluidisation of the catalyst.

The catalyst was activated by passing a stream of dibromodifluoromethane through it and raising the temperature to 350°C. The temperature was then adjusted to about 200°C and dibromodifluoromethane containing between 3% and 10% of bromochlorodifluoromethane was fed into the reactor by means of a peristaltic pump delivering a constant volume of the liquid which was then carried further into the reactor and vapourised by the helium gas stream. The temperature in the reactor was adjusted to the desired reaction temperature and the off-gases were passed through (1) a catchpot at about 30°C, (2) an acid scrubber containing 25% potassium hydroxide solution at about 40°C and (3) a liquid nitrogen receiver at -198°C. The gases leaving the scrubber were analysed by glc.

Details are given in the Table below.

The reactor was a vertically disposed tube made of "Inconel" metal having a bore of 5 cm and a length of 85 cm, there being a 2.5 cm tap to remove spent catalyst and two gas inlet tubes at the lower end. A disengagement space at the top of the tube prevented fine catalyst particles being elutriated into the outlet lines and a 1 cm diameter thermocouple pocket running down the centre of the reactor enabled the temperature of the catalyst to be measured. The reactor tube was heated by an electronically controlled furnace. Trace heating and lagging were provided at the bottom of the reactor and the top disengagement space to prevent the condensation of organic materials.

Example 2

The reactor described in Example 1 was charged with 900 $cm^3$ of a pelletised chromia catalyst prepared as described in our United Kingdom Patent Specification No.1307224. The catalyst was heated to 350°C in a helium gas stream of 200 $cm^3$/min and, after any water present had been carried off, the catalyst was fluorinated in a stream of hydrogen fluoride.

The dibromodifluoromethane feed was then started and the reaction carried out as in Example 1. Details are given in the Table below.

Example 3

The procedure of Example 1 was repeated using a hydrogen fluoride feed of 480 $cm^3$/min together with the dibromodifluoromethane feed. Details are given in the Table below.

Example 4

The procedure of Example 2 was repeated using a hydrogen fluoride feed of 480 $cm^3$/min together with the dibromodifluoromethane feed. Details are given in the Table below.

## TABLE

| Ex | Catalyst | Vol cc | He cc/min | $CF_2Br_2$ cc/min | : total(g) | HF cc/min | : total(g) | TRAPS(g) 1 | 2 | 3 | GAS A | ANALYSIS % B | C | D | TEMP RANGE | %Y A | %Ym A |
|----|----------|--------|-----------|-------|-------|------|------|-----|-----|-----|----|----|----|----|---------|----|----|
| 1 | AlF$_3$ | 800 | 200 | 1.1 | 365 | – | – | 167 | 35 | 191 | 84 | 3 | 3 | 9 | 170–225 | 60 | 89 |
| 2 | Chromia | 900 | 200 | 1.4 | 504 | – | – | 13 | 174 | 289 | 70 | 11 | 7 | 12 | 350–380 | 54 | 82 |
| 3 | AlF$_3$ | 800 | 200 | 1.4 | 391 | 480 | 76 | 13 | 180 | 271 | 86 | 7 | 9 | 2 | 340–360 | 79 | 79 |
| 4 | AlF$_3$ | 800 | 200 | 1.2 | 359 | 330 | 40 | 4 | 152 | 256 | 82 | 5 | – | 6 | 315–345 | 84 | 84 |
| 5 | Chromia | 900 | 200 | 1.8 | 686 | 480 | 76 | 40 | – | 468 | 87 | 5 | – | 4 | 340–365 | 81 | 81 |

Key : Trap 1 is catchpot
Trap 2 is scrubber
Trap 3 is liquid N$_2$ receiver

A is CF$_3$Br
B is CF$_3$Cl
C is CF$_2$BrCl
D is CF$_2$Br$_2$

%Y is conversion of fed material to CF$_3$Br
%Ym is conversion as % of maximum theoretical

The CF$_2$Br$_2$ flow is cc/min of liquid.

CLAIMS

1.      A method for the preparation of bromotrifluoromethane which comprises contacting gaseous dibromodifluoromethane with a fluorination catalyst at a temperature in the range 100-450°C.

2.      A method according to claim 1 wherein a gaseous mixture of dibromodifluoromethane and hydrogen fluoride is contacted with the catalyst at a temperature in the range 100-400°C.

3.      A method according to claim 1 wherein the fluorination catalyst is selected from aluminium fluoride and chromia.

# 0184896

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| D,X | DE-A-1 913 405  (KALI-CHEMIE)<br>* Claims *<br><br>----- | 1,3 | C 07 C   19/00<br>C 07 C   17/00 |

TECHNICAL FIELDS SEARCHED (Int. Cl.4)

C 07 C

The present search report has been drawn up for all claims

| Place of search | Date of completion of the search | Examiner |
|---|---|---|
| THE HAGUE | 06-03-1986 | VAN GEYT J.J.A. |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO Form 1503. 03 82

# EUROPEAN SEARCH REPORT

European Patent Office

Application number

EP 85 30 7403

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| D,X | DE-A-1 913 405 (KALI-CHEMIE) <br> * Claims * <br><br> ----- | 1,3 | C 07 C 19/00 <br> C 07 C 17/00 |
| | | | |
| | | | **TECHNICAL FIELDS SEARCHED (Int. Cl.4)** |
| | | | C 07 C |

The present search report has been drawn up for all claims

| Place of search | Date of completion of the search | Examiner |
|---|---|---|
| THE HAGUE | 06-03-1986 | VAN GEYT J.J.A. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82